Europäisches Patentamt

European Patent Office

Office européen des brevets

(11) Veröffentlichungsnummer: **0 293 890**
**A2**

(12) **EUROPÄISCHE PATENTANMELDUNG**

(21) Anmeldenummer: 88108832.2

(22) Anmeldetag: 02.06.88

(51) Int. Cl.⁴: **A61F 7/00**

(30) Priorität: 04.06.87 DE 3718665

(43) Veröffentlichungstag der Anmeldung:
07.12.88 Patentblatt 88/49

(84) Benannte Vertragsstaaten:
**AT BE CH DE ES FR GB IT LI LU NL SE**

(71) Anmelder: **SANA PLUS GMBH**
**Eichhornstrasse 13**
**D-7750 Konstanz(DE)**

(72) Erfinder: **Baumann, Peter**
**Mozartstrasse 10**
**D-7750 Konstanz(DE)**

(74) Vertreter: **Patentanwälte RUFF, BEIER und**
**SCHÖNDORF**
**Neckarstrasse 50**
**D-7000 Stuttgart 1(DE)**

(54) **Vorrichtung zur Durchführung thermischer Behandlungen des menschlichen Körpers.**

(57) Diese Vorrichtung weist ein den Benutzer aufnehmendes Gehäuse (1) auf, das für den Kopf des Benutzers mit einer Durchstecköffnung (17) versehen ist. Außerdem weist sie eine Heizeinrichtung (23) und schließlich eine Luftaustausch-Einrichtung auf, mittels der das Innere des Gehäuses (1) wahlweise geheizt oder gekühlt werden kann.

FIG. 2

EP 0 293 890 A2

## Vorrichtung zur Durchführung thermischer Behandlungen des menschlichen Körpers

Die Erfindung betrifft eine Vorrichtung zur Durchführung thermischer Behandlungen des menschlichen Körpers mit einem den Benutzer aufnehmenden Gehäuse, das für den Kopf des Benutzers eine Durchstecköffnung aufweist, und einer Heizeinrichtung.

Seit langem sind derartige Vorrichtungen, die zunächst als "Schwitzkästen", später auch als "Heimsaunen" bezeichnet wurden, bekannt. Die Heizeinrichtung dieser Vorrichtungen bestand zunächst aus einem einfachen, mit einer hölzernen Abdeckplatte versehenen Elektroofen, der dem Benutzer zugleich als Sitzgelegenheit diente. Bei neueren Ausführungen erfolgt die Beheizung des Gehäuseinneren durch elektrische Heizelemente, die in der mit Kunststoff kaschierten Gehäusewandung vorgesehen sind, so daß sich der Benutzer im Gehäuseinneren bewegen kann, ohne Gefahr zu laufen, mit heißen Metallflächen in Berührung zu kommen.

Derartige Geräte ermöglichen eine gleichmäßige Durch- bzw. Überwärmung des menschlichen Körpers und eine Beschleunigung des menschlichen Stoffwechsels, der zu einer stärkeren Haut- und Muskeldurchblutung und Schweißausonderung fürt, weshalb diese Geräte heute insbesondere zur Haut- und Schönheitspflege angeboten werden. Sie weisen jedoch aufbaubedingte Nachteile auf, die insbesondere von älteren oder weniger gelenkigen Benutzern als nachteilig empfunden werden. Ihre Gehäuse, die im wesentlichen aufrechtstehenden Quadern oder Zylindern entsprechen, weisen nämlich eine von oben aufsetzbare Oberseite mit einer Durchstecköffnung für den Kopf des Benutzers auf, die der Benutzer vor Behandlungsbeginn mit der vertikalen Gehäusewandung verbinden muß, was ihm oft allein nicht möglich ist.

Der Erfindung liegt die Aufgabe zugrunde, die Einsatzmöglichkeiten und die Handhabung einer Vorrichtung der eingangs beschriebenen Art zu verbessern.

Diese Aufgabe wird erfindungsgemäß dadurch gelöst, daß eine Luftaustausch-Einrichtung zur Kühlung des Gehäuseinneren vorgesehen ist. Diese Luftaustausch-Einrichtung bietet dem Benutzer nicht nur die Möglichkeit, warm-kalte "Wechselbäder" durchzuführen. Sie macht es vielmehr auch möglich, die Luftfeuchtigkeit im Gehäuseinneren zu reduzieren, so daß Schweißausdünstungen rasch vertrocknen und als weniger störend empfunden werden.

Bei der erfindungsgemäßen Vorrichtung ist mit Vorteil ein auf das Gehäuseinnere einwirkendes Gebläse vorgesehen. Dieses Gebläse kann als Druck- oder Sauggebläse ausgebildet, d.h. so ausgelegt sein, daß es entweder Luft in das Gehäuseinnere drückt oder Luft aus dem Gehäuseinneren saugt und bietet neben der Möglichkeit eines raschen Luftaustausches den Vorteil, daß die Einrichtung zur Beheizung des Gehäuseinneren gewünschtenfalls auch außerhalb des Gehäuses angeordnet sein kann. Vorzugsweise ist ein Gebläse vorgesehen, das außerhalb des Gehäuses erzeugte Warmluft in das Gehäuseinnere drückt. Dieses oder ein anderes Gebläse kann dazu vorgesehen sein, außerhalb des Gehäuses vorhandene Kaltluft anzusaugen und in das Gehäuseinnere zu drücken. Zur Temperaturänderung im Gehäuseinneren kann ein wahlweise Warm- oder Kaltluft förderndes Gebläse vorgesehen sein.

Das dem Luftaustausch und/oder der Temperaturänderung innerhalb des Gehäuses dienende Gebläse ist mit Vorteil außerhalb des Gehäuses angeordnet und arbeitet vorzugsweise im Umluftbetrieb, wobei ihm vorzugsweise wenigstens ein Misch- bzw. Umschaltventil zugeordnet ist, so daß der von ihm geförderten Luft wahlweise ein mehr oder weniger großer Anteil an Frischluft zugeführt werden kann.

Bei einer bevorzugten Ausführungsform ist die Vorrichtung so eingerichtet, daß die Luft stets in das Gehäuse geblasen wird. Der schwache Warmluftstrom vermag dabei in der Gehäusewandung vorgesehene gewichts- oder kraftbelastete Entlüftungsklappen, nicht oder nur wenig zu öffnen. Ein zur Kaltluftdusche verwendeter starker unbeheizter Luftstrom öffnet durch erhöhten Druck die Entlüftungsklappen, die somit einen starken Luftdurchsatz und eine schnelle Abkühlung ermöglichen. Bei einer anderen, ebenfalls vorteilhaften Ausführungsform sind die Klappen als Ansaug- bzw. Rückschlagklappen ausgebildet. Warmluft wird langsam eingeblasen, wobei die Rückschlagklappen ein Entweichen der Warmluft nur wenig oder nicht ermöglichen. Zur Kühlung wird die Luft zum Beispiel durch mechanische Umlenkung oder Änderung der Motorförderrichtung reversiert. Dadurch wird die warme Luft aus dem Gehäuse abgesaugt und durch Frischluft ersetzt, die durch die jetzt geöffneten Klappen ungehindert einströmt.

Dem bzw. den auf das Gehäuseinnere einwirkenden Gebläse kann jeweils mit Vorteil eine Luftentkeimungs-Einrichtung und/oder ein Luftfilter und/oder eine Luftbefeuchtungs-Einrichtung und/oder eine Luftentfeuchtungs-Einrichtung zugeordnet sein, so daß die Beschaffenheit der im Gehäuseinneren vorhandenen Luft entsprechend beeinflußt werden kann.

Die Gehäuseoberseite der erfindungsgemäßen Vorrichtung weist wie auch die bisher bekannten Vorrichtungen der eingangs beschriebenen Art eine für den Kopf des Benutzers vorgesehene Durchstecköffnung auf. Sie verläuft jedoch in Sichtrichtung des Benutzers abwärts, bei einer bevorzugten Ausführungsform im wesentlichen parallel zur Verbindungslinie zwischen den Schultern und den Knien der im Gehäuseinneren sitzenden Person, wodurch einerseits das Blickfeld des Benutzers erweitert und andererseits das Volumen des Gehäuseinneren verkleinert wird. Die Blickfelderweiterung führt dazu, daß der Vorrichtungsbenutzer mehr am Umweltgeschehen teilnehmen, d.h. beispielsweise während der Vorrichtungsbenutzung fernsehen kann. Die Verringerung des Gehäusevolumens bietet insbesondere den Vorteil, daß zur Beheizung und/oder zur Kühlung des Gehäuseinneren weniger Energie aufgebracht werden muß. Unabhängig davon lassen sich bei geringerem Luftvolumen "Wechselbäder" rascher durchführen.

Abgesehen von der schrägen Ausrichtung seiner Oberseite kann das Gehäuse der erfindungsgemäßen Vorrichtung grundsätzlich beliebig, d.h. in Draufsicht beispielsweise quadratisch, rechteckig oder kreisförmig ausgebildet sein, d.h. in seiner äußeren Form einen schräg geschnittenen Würfel, einem schräg geschnittenen Quader oder einem - schräg geschnittenen Zylinder entsprechen. Als vorteilhaft hat sich jedoch eine Gehäuseform erwiesen, bei der zumindest die Rückseite in Draufsicht bogenförmig verläuft und im Bereich der zentralen Mantellinie, die in der vertikal verlaufenden Gehäuse-Symmetrieebene liegt, die höchste Höhe aufweist.

Das Gehäuse der erfindungsgemäßen Vorrichtung ist vorzugsweise zweiteilig ausgebildet, so daß es auch vom Laien jederzeit problemlos zerlegt werden kann.

Bei einer bevorzugten Ausführungsform weist das Gehäuse der erfindungsgemäßen Vorrichtung einen in Vorderansicht im wesentlichen rechteckigen, vertikal verlaufenden Vorderwandbereich auf, der mit der Gehäuseoberseite vorzugsweise verbunden ist, wobei die Verbindung zwischen diesem Vorderwandbereich und der Gehäuseoberseite vorzugsweise gelenkartig ausgebildet ist. Das Gehäuse kann dabei einen Boden aufweisen, der mit dem vorgenannten, in Vorderansicht im wesentlichen rechteckigen, vertikal verlaufenden Vorderwandbereich vorzugsweise ebenfalls verbunden ist. Die Gehäuseoberseite kann dabei mit Vorteil einen die im montierten Zustand bogenförmig verlaufende Gehäusewandung außen umgreifenden Randbereich aufweisen. Auch der Gehäuseboden kann einen der im montierten Zustand bogenförmig verlaufenden Gehäusewandung außen anliegenden Wulst aufweisen, so daß die Verbindung zwischen der bogenförmig verlaufenden Gehäusewandung sowohl mit der Gehäuseoberseite als auch mit dem Gehäuseboden problemlos vorgenommen werden kann.

Zur Betätigung des bzw. der Gebläse und der gewünschtenfalls zugeordneten Aggregate kann innerhalb des Gehäuses der erfindungsgemäßen Vorrichtung eine entsprechende Umschalteinrichtung vorgesehen sein, die für den Vorrichtungsbenutzer den Vorteil bietet, unabhängig von Dritten zu sein.

Die für den Kopf des Benutzers im Gehäuseoberteil vorgesehene Durchstecköffnung kann in eine beispielsweise durch Druckknöpfe oder einen Reißverschluß verschließbare Öffnung übergehen, die den Vorteil bietet, daß die Gehäuseoberseite beim Ein- und Ausstieg nicht von der Gehäuseseitenwand gelöst zu werden braucht.

An der Gehäuseoberseite kann im sichtbaren Bereich eine Anzeigetafel angebracht sein, mit Kontrollinstrumenten, wie einem Thermometer, einem Hygrometer, einer Zeit- bzw. Stoppuhr.

Ebenfalls an der Gehäuseoberseite kann eine Verdunstungseinrichtung für Inhalationsmedien vorgesehen sein, wobei die Wärme im Gehäuseinneren zur Verdunstung ausnutzbar ist, insbesondere durch die geschlossene Wandung hindurch. Durch einen kamin- bzw. tunnelartigen Aufsatz kann eine thermische Luftströmung erzeugt werden, die zum Gesicht des Benutzers gerichtet ist und dabei die Inhalationsstoffe aufnimmt und abgibt.

Weitere Einzelheiten, Merkmale und Vorteile der Erfindung, die für sich allein oder zu mehreren in Kombination bei einer Ausführungsform der Erfindung verwirklicht sein können, ergeben sich aus den Ansprüchen und der nachfolgenden Beschreibung einer bevorzugten Ausführungsform der Erfindung im Zusammenhang mit der Zeichnung.

In der Zeichnung zeigen jeweils schematisch:

Fig. 1 eine Seitenansicht einer Vorrichtung gemäß der Erfindung;

Fig. 2 die Draufsicht der in Fig. 1 dargestellten Vorrichtung;

Fig. 3 Seitenwand und Boden der in den Fig. 1 und 2 dargestellten Vorrichtung vor ihrer Montage in verkleinertem Maßstab.

Die in der Zeichnung schematisch dargestellte Vorrichtung zur Durchführung thermischer Behandlungen des menschlichen Körpers weist ein zweiteiliges, vorzugsweise aus bzw. unter Verwendung von Kunststoff gefertigtes Gehäuse 1 auf, das aus einem Gehäuseboden 2, einer Gehäuseseitenwand 3, 4 und einer Gehäuseoberseite 5 besteht, wobei der Gehäuseboden 2, ein Bereich 3 der Gehäuseseitenwand und die Gehäuseoberseite 5 einstückig ausgebildet sind.

Die Gehäuseoberseite 5, der Vorderwandbereich 3 und der Gehäuseboden 2 bestehen aus flexiblem Material, die übrigen Teile aus selbsttragendem Material.

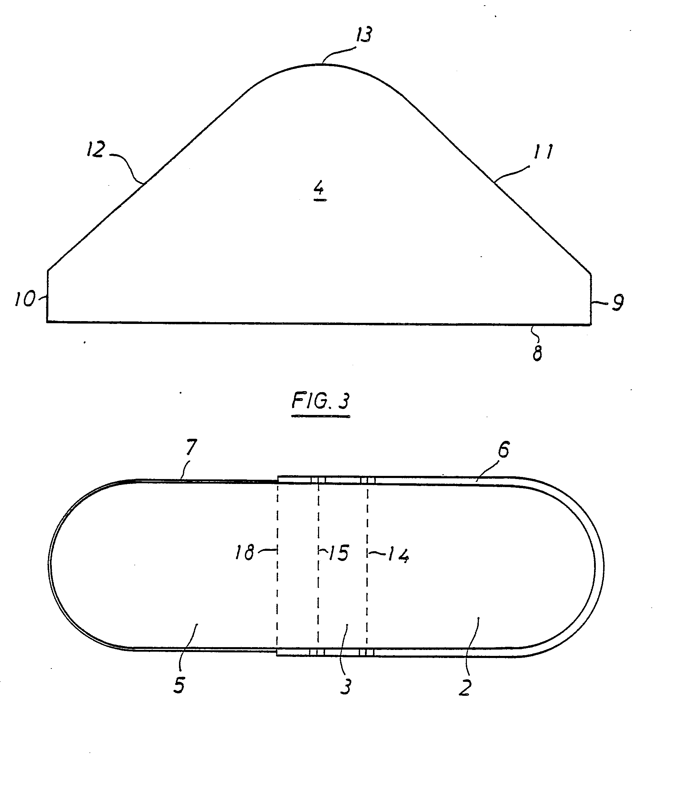

Die Form des Gehäusebodens 2 entspricht in Draufsicht der eines Rechteckes, dessen eine Schmalseite halbkreisbogenförmig verläuft. An der gegenüberliegenden Schmalseite des Bodens 2 ist der vorerwähnte, rechteckig ausgebildete Seitenwandbereich 3 und an diesen die Gehäuseoberseite 5 angelenkt, die an ihrer von ihrer Anlenkung abgewandten Seite bogenförmig ausgebildet ist. Breitet man die aus dem Gehäuseboden 2, dem Bereich 3 der Gehäusewand und der Gehäuseoberseite 5 gebildete Einheit eben aus, wie dies in Fig. 3 unten dargestellt ist, so ergibt sich demnach ein im wesentlichen rechteckiges langgestrecktes Gebilde, dessen beide Schmalseiten bogenförmig ausgebildet sind. Der rechteckige Seitenwandbereich 3 und der Gehäuseboden 2 sind von einem nach oben weisenden, relativ dicken Wulst 6, die Gehäuseoberseite 5 von einem in dieser Lage ebenfalls nach oben weisenden Rand 7 umgeben.

Die Gehäusewand 4 weist bei der in Fig. 3 oben dargestellten planen Ausrichtung eine relativ lange, im montierten Zustand horizontal verlaufende Seitenkante 8 auf, an deren Enden sich gleich kurze, im montierten Zustand vertikal verlaufende Seitenkanten 9, 10 anschließen. An den oberen Enden dieser Seitenkanten 9, 10 schließen sich konvergierende Schrägkanten 11, 12 an, die oberhalb der Mitte der Seitenkante 8 über einen bogenförmigen Kantenbereich 13 ineinander übergehen.

Die im montierten Zustand horizontal verlaufende Seitenkante 8 entspricht in ihrer Länge der Summe der Längen der Längsseiten und der bogenförmigen Schmalseite des Gefäßbodens 2, die Länge der im montierten Zustand vertikalen Seitenkanten 9, 10 der Breite des mit dem Gefäßboden 2 verbundenen, seitlich durch Knickkanten 14, 15 begrenzten Seitenwandbereiches 3 und der größte Abstand des bogenförmigen Kantenbereiches 13 der Gehäuseseitenwand 4 von deren langer Seitenkante 8 dem größten Abstand der bogenförmig verlaufenden Kante des Gefäßoberteiles 5 von der vorgenannten Knickkante 15. Die Gehäuseseitenwand 4 und die aus dem Gehäuseboden 2, der Gehäuseseitenwand 3 und dem Gehäuseoberteil 5 gebildete Einheit sind also in ihren Abmessungen so aufeinander abgestimmt, daß sie auch ohne Zuhilfenahme spezieller Werkzeuge vom Laien problemlos miteinander verbunden werden können. Die Gehäuseseitenwand 4 ist hierzu zunächst so zu verbiegen, daß ihre lange Seitenkante 8 in Draufsicht U-bogenförmig verläuft. In diesem Zustand ist die Gehäuseseitenwand 4 auf den Gehäuseboden 2 aufzusetzen und zwar so, daß der Wulst 6 außen rundum an der Gehäuseseitenwand 4 anliegt. Sodann ist der mit dem Gehäuseboden 2 über die Knickkante 14 verbundene Gehäusewandbereich 3 rechtwinklig nach oben zu biegen und schließlich der mit dem Gehäusewandbereich 3 über die Knickkante 15 verbundene Gehäuseoberteil 5 so weit in Richtung auf die jetzt ebenfalls in Draufsicht U-bogenförmig verlaufende Oberkante der Gehäuseseitenwand 4 zu biegen, daß ihr Rand 7 auen an den oberen Bereich der Gehäuseseitenwand 4 zur Anlage gebracht werden kann. Um ein Knicken des Gehäusewandbereiches 3 und des damit verbundenen Gehäuseoberteiles 5 zu gewährleisten, weist der im Gehäusewandbereich 3 und zunächst auch den Gehäuseoberteil 5 seitlich begrenzenden Wulst 6 im Bereich der Knickkanten 14, 15 entsprechende Einkerbungen auf.

Ist der Gehäuseoberteil 5 über seinen Rand 7 mit der Oberkante der Gehäuseseitenwand 4 verbunden, so stellt das damit fertig montierte Gehäuse ein pultartig ausgebildetes stabiles Gehäuse dar, dessen Grundriß einem Rechteck entspricht, dessen eine Schmalseite kreisbogenförmig verläuft. Zugänglich ist dieses Gehäuse durch eine verschließbare Öffnung 16 im Gehäuseoberteil 5, die an ihrem oberen Ende in eine im wesentlichen kreisförmige Durchstecköffnung 17 für den Kopf des Benutzers übergeht oder aber dadurch, daß das Gehäuseoberteil 5 von der Gehäuseseitenwand 4 abgehoben und um eine hierzu vorgesehene Knickkante 18 umgeschlagen wird.

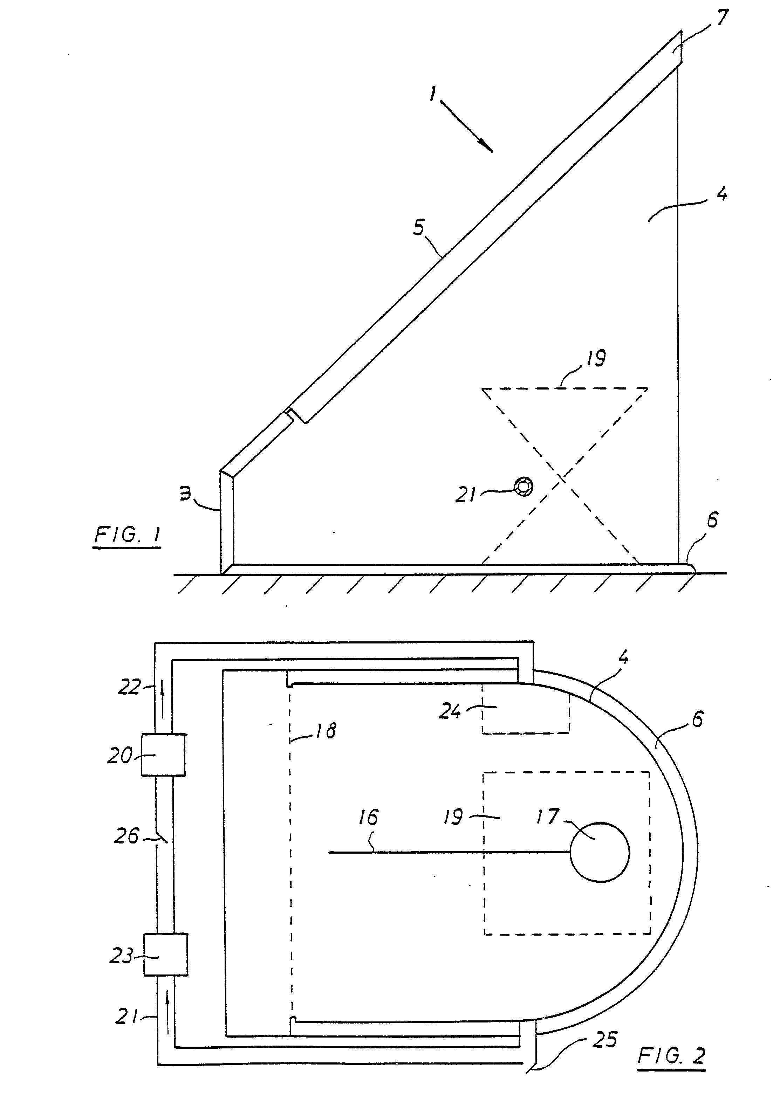

Im Inneren des Gehäuses 1 kann für den Benutzer eine Sitzgelegenheit 19, beispielsweise ein Hocker oder ein Klappstuhl, untergebracht werden, wie dies in den Fig. 1 und 2 schematisch dargestellt ist. Außerhalb des Gehäuses (vgl. Fig. 2) ist ein von einem Elektromotor angetriebenes Gebläse 20 angeordnet, das beidseitig über Leitungen 21, 22 mit dem Inneren des Gehäuses 1 verbunden ist. Das Gebläse 20 ist so ausgelegt, daß es in Richtung der in Fig. 2 dargestellten Pfeile im Umluftbetrieb arbeitet. Ihm ist ein elektrisches Heizaggregat 23 vorgeschaltet, das, wie das Gebläse 20 auch, vom Benutzer ein- und ausgeschaltet werden kann. Zum Betätigen des Gebläses 20 und/oder des ihm vorgeschalteten Heizaggregates 23 sind Schalter vorgesehen, die im Inneren des Gehäuses 1 an einem vom Benutzer problemlos erreichbaren Schaltpult 24 angeordnet sind. Die Leitungen 21 und 22 weisen Ventilklappen 25, 26 auf, die vom Schaltpult 24 aus ebenfalls betätigt werden können. Vom Schaltpult 24 aus können schließlich auch in der Zeichnung nicht dargestellte Einrichtungen betätigt werden, mit denen die Leitungen 21 und 22 in Wirkverbindung gebracht werden können, wie beispielsweise eine Luftentkeimungs-Einrichtung, ein Luftfilter oder Einrichtungen zur Luftbe- bzw. -entfeuchtung.

Hat der Vorrichtungsbenutzer im Inneren des Gehäuses 1 Platz genommen und ist die Gehäuseoberseite 5 dicht mit der Gehäuseseitenwand 4 verbunden, so werden zunächst die Ventile 25, 26 geschlossen. Dann wird das Heizaggregat 23 und gleichzeitig das ihm nachgeordnete Gebläse 20 eingeschaltet. Das Gebläse 20 saugt jetzt Luft aus dem Inneren des Gehäuses 1 an, die vom Heizaggregat 23 erwärmt und dann dem Gebläse 20 zugeführt wird, um dann in das Innere des Gehäuses 1 gedrückt zu werden. Die Lufttemperatur im Inneren des Gehäuses 1 steigt, bis sie die vom Benutzer gewünschte Höhe erreicht hat. Wünscht der Benutzer eine Abkühlung, so ist dies problemlos und auch relativ rasch möglich. Hierzu brauchen nur die beiden Ventile 25 und 26 geöffnet und das Heizaggregat 23 abgeschaltet zu werden. Das Gebläse 20 saugt jetzt Frischluft aus dem ihm umgebenden Raum über das Ventil 26 an und drückt diese über die Leitung 22 in das Innere des Gehäuses 1, während ein Teil der unerwünschten Warmluft aus dem Gehäuse 1 über das Ventil 25 ins Freie gelangt.

Wird nicht im Umluftbetrieb gearbeitet, dann kann die Rückführleitung 21 weggelassen und beim Heizaggregat 23 durch einen Frischluft-Ansatzstutzen ersetzt werden. Die Ventilklappe 25 bleibt jedoch am Gehäuseauslaßstutzen.

Bei Warmluftbetrieb mit geringem Druck bleibt die Klappe 25 geschlossen, so daß der Luftdurchsatz nur durch normale Undichtigkeiten im Gehäuse erfolgt. Bei Kaltluftzustrom mit erhöhtem Druck öffnet sich die Klappe 25 und ermöglicht einen erhöhten Luftdurchsatz. Wird die Abkühlung durch Luftabsaugung, zum Beispiel durch Umkehr der Förderrichtung des Motors erzeugt, dann ist die Ventilklappe 25 als nach innen öffenbare Rückschlagklappe ausgebildet.

## Ansprüche

1. Vorrichtung zur Durchführung thermischer Behandlungen des menschlichen Körpers mit einem den Benutzer aufnehmenden Gehäuse, das für den Kopf des Benutzers eine Durchstecköffnung aufweist, und einer Heizeinrichtung, dadurch gekennzeichnet, daß eine Luftaustausch-Einrichtung zur Kühlung des Gehäuseinneren vorgesehen ist.

2. Vorrichtung nach Anspruch 1, dadurch gekennzeichnet, daß ein auf das Gehäuseinnere einwirkendes, vorzugsweise wahlweise Warmluft oder Kaltluft förderndes Gebläse (20) vorgesehen ist, das vorzugsweise außerhalb des Gehäuses (1) angeordnet ist.

3. Vorrichtung nach Anspruch 1 oder 2, dadurch gekennzeichnet, daß ein Druckgebläse vorgesehen ist, das Warmluft und vorzugsweise alternativ Kaltluft zu fördern vermag, letzteres insbesondere bei erhöhter Drehzahl.

4. Vorrichtung nach einem der vorhergehenden Ansprüche, dadurch gekennzeichnet, daß ein Gebläse vorgesehen ist, das für Kaltluftförderung als Sauggebläse, insbesondere mit erhöhter Drehzahl, und für Warmluftförderung als Druckgebläse, insbesondere bei niedriger Drehzahl, ausgebildet ist.

5. Vorrichtung nach einem der Ansprüche 2 bis 4, dadurch gekennzeichnet, daß das Gebläse (20) ein im Umluftbetrieb arbeitendes Gebläse ist.

6. Vorrichtung nach einem der Ansprüche 2 bis 5, dadurch gekennzeichnet, daß dem Gebläse wenigstens ein Mischbzw. Umschaltventil zugeordnet ist.

7. Vorrichtung nach einem der Ansprüche 2 bis 6, dadurch gekennzeichnet, daß dem Gebläse eine Luftentkeimungs-Einrichtung, ein Luftfilter, eine Luftbefeuchtungs-Einrichtung und/oder eine Luftentfeuchtungs-Einrichtung zugeordnet ist.

8. Vorrichtung nach einem der vorhergehenden Ansprüche, dadurch gekennzeichnet, daß die Luftaustauscheinrichtung pneumatisch öffenbare Verschlußklappen in der Gehäusewandung aufweist.

9. Vorrichtung zur Durchführung thermischer Behandlungen des menschlichen Körpers mit einem den Benutzer aufnehmenden Gehäuse, das für den Kopf des Benutzers eine Durchstecköffnung und in seinem Inneren eine Sitzgelegenheit aufweist, sowie einer Einrichtung zur Beheizung des Gehäuseinneren, insbesondere nach einem der vorhergehenden Ansprüche, dadurch gekennzeichnet, daß die für den Kopf des Benutzers vorgesehene Durchstecköffnung (17) in an sich bekannter Weise in der Gehäuseoberseite (5) vorgesehen ist, und daß die Gehäuseoberseite (5) schräg, in Sichtrichtung des Benutzers abwärts verläuft, wobei die für den Kopf des Benutzers vorgesehene Durchstecköffnung (17) vorzugsweise in eine verschließbare Öffnung (16) des Gehäuseoberteiles (5) übergeht.

10. Vorrichtung nach Anspruch 9, dadurch gekennzeichnet, daß zumindest die Gehäuserückseite in Draufsicht bogenförmig verläuft und zumindest im Bereich der zentralen Mantellinie die höchste Gehäusehöhe aufweist, insbesondere das Gehäuse (1) eine selbsttragende transportable Seitenwand (4) aufweist, die im montierten Zustand im Querschnitt U-bogenförmig um die im Gehäuse (1) sitzende Person verläuft.

11. Vorrichtung nach Anspruch 9 oder 10, dadurch gekennzeichnet, daß das Gehäuse (1) zweiteilig ausgebildet ist, vorzugsweise ein die Gehäuseoberseite (5) und den Boden umfassendes Teil und ein Wandbereiche umfassendes Teil aufweist.

12. Vorrichtung nach einem der Ansprüche 9 bis 11, dadurch gekennzeichnet, daß das Gehäuse (1) einen in Vorderansicht im wesentlichen rechteckigen, vertikal verlaufenden Wandbereich (3) aufweist, der mit der Gehäuseoberseite (5) vorzugsweise verbunden, insbesondere einstückig ausgebildet ist und einen Boden (2), der mit dem in Vorderansicht im wesentlichen rechteckigen, vertikal verlaufenden Seitenwandbereich (3) vorzugsweise verbunden ist.

13. Vorrichtung nach einem der vorhergehenden Ansprüche, dadurch gekennzeichnet, daß die Gehäuseoberseite (5) einen die im montierten Zustand bogenförmig verlaufende Gehäusewand (4) außen umgreifenden Randbereich (7) aufweist und vorzugsweise der Boden (2) einen an der im montierten Zustand bogenförmig verlaufenden Gehäusewand> (4) außen anliegenden Wulst (6) aufweist.

14. Vorrichtung nach einem der Ansprüche 2 bis 13, dadurch gekennzeichnet, daß zur Betätigung des Gebläses innerhalb des Gehäuses (1) eine Umschalteinrichtung vorgesehen ist.

15. Vorrichtung nach einem der vorhergehenden Ansprüche, dadurch gekennzeichnet, daß der Gehäuseoberseite eine Einrichtung zum Verdunsten von Inhalationsmedien zugeordnet ist, die vorzugsweise durch die Wärme im Gehäuseinneren indirekt beheizbar ist.

B1.

_FIG. 1_

_FIG. 2_

13

12    4    11

10    9

8

FIG. 3

7    6

18    15    14

5    3    2